(19) Europäisches Patentamt
European Patent Office
Office européen des brevets

(11) Veröffentlichungsnummer: **0 226 924**
**A1**

(12) # EUROPÄISCHE PATENTANMELDUNG

(21) Anmeldenummer: 86117022.3

(22) Anmeldetag: 08.12.86

(51) Int. Cl.⁴: A 61 K 6/10
C 08 L 35/00

(30) Priorität: 21.12.85 DE 3545608

(43) Veröffentlichungstag der Anmeldung:
01.07.87 Patentblatt 87/27

(84) Benannte Vertragsstaaten:
AT BE CH DE FR GB IT LI NL SE

(71) Anmelder: BAYER AG
Konzernverwaltung RP Patentabteilung
D-5090 Leverkusen 1 Bayerwerk(DE)

(72) Erfinder: Voigt, Reiner, Di.
Gustav-Radbruch-Strasse 14
D-5090 Leverkusen 3(DE)

(72) Erfinder: Schuster, Klaus, Di.
Gerhart-Hauptmann-Strasse 33
D-5090 Leverkusen 3(DE)

(72) Erfinder: Bartl, Herbert, Dr.
Eichendorffweg 10
D-5068 Odenthal(DE)

(72) Erfinder: Schwabe, Peter, Dr.
Dudweiler Strasse 17
D-5090 Leverkusen 1(DE)

(54) **Abformmassen.**

(57) Abformmassen, bevorzugt für den Dentalbereich, enthalten Copolymere mit der Struktureinheit

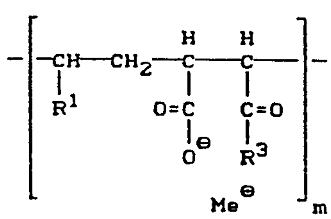

EP 0 226 924 A1

Croydon Printing Company Ltd.

0226924

BAYER AKTIENGESELLSCHAFT 5090 Leverkusen, Bayerwerk
Konzernverwaltung RP
Patentabteilung Mn/by-c

## Abformmassen

Die Erfindung betrifft Abformmassen auf Basis von Copolymeren, vorzugsweise für dentale Anwendungszwecke.

Im Rahmen der vorliegenden Erfindung werden Abformmassen zur Herstellung von Abdrücken von Gegenständen verwendet, von denen ein räumliches Abbild hergestellt werden soll. Die Abformmassen (auch Dubliermassen) können, bevorzugt im Dentalbereich bei der Herstellung von Inlays, Kronen, Brücken und Prothesen eingesetzt werden. Mit ihrer Hilfe kann ein Negativ der Kiefernsituation hergestellt werden, welches anschließend, z.B. mit Modellgips, ausgegossen wird. Das so erhaltene Modell wird als Grundlage für die weiteren Arbeiten beim Zahntechniker verwandt.

Es sind eine Reihe von Materialien für Abformmassen bekannt, z.B. Polysiloxane, Polysulfide, Polyether, reversible und irreversible Hydrokolloide, insbesondere Alginate.

Abdruckmassen auf Alginatbasis sind z.B. aus der US 23 45 255, US 23 90 137, US 23 97 145, US 24 22 487, US 27 33 156, US 28 78 129, US 32 46 998, GB 51 8596, DE-OS 25 11 168 und DE-OS 31 35 567 bekannt.

Abformmaterialien auf Alginatbasis werden unmittelbar vor der Anwendung mit Wasser oder einer wasserhaltigen Komponente versetzt und zu einer Paste verrührt, die direkt zur Herstellung des Abdrucks verwendet wird. Die bekannten Alginatabformmassen schrumpfen durch Verdunsten des Wassers und bleiben daher, insbesondere bei längerer Lagerung, nicht formstabil. Außerdem werden die Alginate durch Bakterien abgebaut, so daß nach einiger Zeit sich die Pasten- und Gummieigenschaften verändern.

Es wurden neue Abdruckmassen gefunden, die Copolymere mit der Struktureinheit

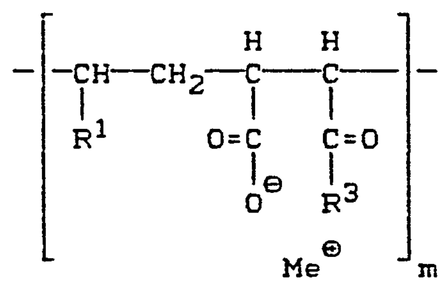

(I),

in der

$Me^{\oplus}$ für Wasserstoff, ein Alkali- oder ein Ammoniumkation steht,

$R^1$ Wasserstoff oder gegebenenfalls substituiertes Alkyl, Aryl, Heterocyclyl oder die Gruppen

$$-O-R^2 \qquad \text{oder} \qquad -O-\overset{O}{\overset{\|}{C}}-R^2$$

in denen

$R^2$ für Alkyl steht,

bedeutet,

$R^3$ ein Rest aus der Gruppe

$-O^{\ominus}Me^{\oplus}$, $-O-R^4$, $-NH_2$, $-NHR^4$, 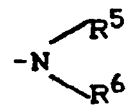 , $-NH-R^7-OH$,

$-NH-R^7-SO_3^{\ominus}Me^{\oplus}$, $-NH-\overset{O}{\overset{\|}{C}}-NH_2$, $-NH-R^7-\overset{O}{\overset{\|}{C}}-O^{\ominus}Me^{\oplus}$ oder

$$\left[-O-\overset{R^8}{\overset{|}{CH}}-CH_2-\right]_n OR^9$$

in denen

$Me^{\oplus}$ die obengenannte Bedeutung hat,

$R^4$,$R^5$,$R^6$ gleich oder verschieden sind und Alkyl oder einen Etherrest bedeuten,

wobei $R^5$ und $R^6$ über Alkylen oder einen zweibindigen Etherrest zu einem 5- oder 6-gliedrigen Ring verbunden sein können,

$R^7$ Alkylen bedeutet und

$R^8$ und $R^9$ gleich oder verschieden sind und Wasserstoff oder Niederalkyl bedeuten und

n eine Zahl von 1 bis etwa 2000 bedeutet, und

m im Bereich von etwa $10^2$ bis etwa $10^6$ liegt,

und Salze mehrwertiger Metalle enthalten.

Die erfindungsgemäßen Abdruckmassen sind gegenüber herkömmlichen Hydrokolloiden bei der Lagerung formstabil und werden nicht durch Bakterien zersetzt.

Alkalikationen können Kationen von Metallen der ersten Gruppe des Periodensystems nach Mendelejew sein. Vorzugsweise seien Natrium- und Kaliumkationen genannt.

Ammoniumkationen können erfindungsgemäß Ammonium oder die Kationen mono-, di-, tri- und/oder tetrasubstituierter Alkyl($C_1$ bis $C_6$)amine sein, wobei die Alkylreste durch Hydroxy substituiert sein können. Erfindungsgemäß werden Ammonium, Triethylammonium und Triethanolammonium bevorzugt.

Alkyl bedeutet erfindungsgemäß im allgemeinen einen geradkettigen oder verzweigten Kohlenwasserstoffrest mit 1 bis 8 Kohlenstoffatomen. Bevorzugt wird Niederalkyl mit 1 bis etwa 6 Kohlenstoffatomen. Beispielsweise seien die folgenden Alkylreste genannt: Methyl, Ethyl, Propyl, Isopropyl, Butyl, Isobutyl, Pentyl, Isopentyl, Hexyl, Isohexyl, Heptyl, Isoheptyl, Octyl und Isooctyl.

Aryl bedeutet im allgemeinen erfindungsgemäß einen aromatischen Rest mit 6 bis 12 Kohlenstoffatomen. Bevorzugt seien Phenyl, Naphthyl und Biphenyl genannt.

Heterocyclyl bedeutet im allgemeinen erfindungsgemäß einen aromatischen Rest mit 4 bis 10 Kohlenstoffatomen und 1 oder 2 Sauerstoff- und/oder Stickstoffatomen im Ring. Bevorzugt sei Pyrrolidyl, Pyridyl und Caprolactyl genannt.

Die Alkyl-, Aryl- und Heterocyclylreste können beispielsweise durch Niederalkyl ($C_1$ bis etwa $C_6$), und Niederalkoxy ($C_1$ bis etwa $C_6$) substituiert sein.

Etherreste bedeuten im allgemeinen erfindungsgemäß einen 1 bis 3 Sauerstoffatome enthaltenden geradkettigen oder verzweigten Kohlenwasserstoffrest mit 1 bis 8 Kohlenstoffatomen. Beispielsweise seien die folgenden Etherreste genannt: $-CH_2-O-CH_3$, $-CH_2-O-CH_2-CH_2-O-CH_3$ und

$-CH_2-O-CH-CH_2-OCH_3$
|
$CH_3$

Alkylen bedeutet im allgemeinen erfindungsgemäß einen zweibindigen geradkettigen oder verzweigten Kohlenwasserstoffrest ($C_4$ bis $C_7$). Beispielsweise seien die folgenden Alkylenreste genannt: Butylen, Pentylen, iso-Pentylen und iso-Hexylen.

Zweibindige Etherreste können im allgemeinen geradkettige oder verzweigte Kohlenwasserstoffreste ($C_3$ bis $C_6$) mit einem oder zwei Sauerstoffatomen sein. Beispielsweise seien die folgenden Reste genannt: $-CH_2-O-CH_2-CH_2-$, $-CH_2-CH_2-O-CH_2-CH_2$ -.

$R^5$ und $R^6$ können über die Alkylen- oder die zweibindigen Etherreste zu einem 5- oder 6-gliedrigen Ring verbunden sein. Beispielsweise seien hier die folgenden Ringsysteme genannt:

Morpholinyl, Piperidyl.

Alkylen ($R^7$) bedeutet im allgemeinen erfindungsgemäß einen 2-bindigen geradkettigen oder verzweigten Kohlenwasserstoffrest mit 1 bis 8, bevorzugt 1 bis 6, insbesondere bevorzugt 1 oder 2, Kohlenstoffatomen. Bevorzugt seien Methylen und Ethylen genannt.

Die Zahl n kann erfindungsgemäß Werte von 1 bis etwa 2000, bevorzugt Werte von 1 bis etwa 400, annehmen.

Die Zahl m liegt erfindungsgemäß im Bereich von etwa $10^2$ bis etwa $10^6$, bevorzugt $10^3$ bis $10^5$.

Erfindungsgemäß werden Copolymere mit der Struktureinheit nach Formel (I) bevorzugt, in der

$Me^{\ominus}$ für Wasserstoff, ein Alkali- oder ein Ammoniumkation steht,

$R^1$ Wasserstoff oder gegebenenfalls substituiertes Alkyl (1 bis 8 Kohlenstoffatome), Aryl (6 bis 12 Kohlenstoffatome und 1 oder 2 Sauerstoff und/oder Stickstoff) oder die Gruppen

$-O-R^2$ oder $-O-\overset{\overset{\displaystyle O}{\|}}{C}-R^2$

in denen

$R^2$ für Alkyl (1 bis 8 Kohlenstoffatome) steht,

bedeutet,

$R^3$ ein Rest aus der Gruppe

$-O^{\ominus}Me^{\oplus}$, $-O-R^4$, $-NH_2$, $-NHR^4$, 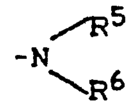 , $-NH-R^7-OH$,

$-NH-R^7-SO_3^{\ominus}Me^{\oplus}$, 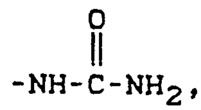, 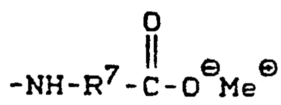 oder

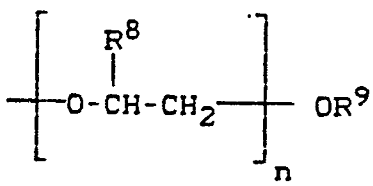

in der

$Me^{\oplus}$ die obengenannte Bedeutung hat,

$R^4$,$R^5$,$R^6$ gleich oder verschieden sind und Alkyl (1 bis 8 Kohlenstoffatome) oder einen Etherrest (1 bis 8 Kohlenstoffatome und 1 bis 3 Sauerstoffatome) bedeuten, wobei $R^5$ und $R^6$ über Alkylen oder einen zweibindigen Etherrest zu einem 5- oder 6-gliedrigen Ring verbunden sein können,

$R^7$ Alkylen mit 1 bis 8 Kohlenstoffatomen bedeutet,

$R^8$ und $R^9$ gleich oder verschieden sind und Wasserstoff oder Niederalkyl bedeuten und

n eine Zahl von 1 bis etwa 2000 bedeutet, und

m im Bereich von etwa $10^2$ bis etwa $10^6$ liegt.

Insbesondere bevorzugt werden erfindungsgemäß Copolymere mit der Struktureinheit nach Formel (I), in der

$Me^{\ominus}$ für Wasserstoff, ein Alkali- oder ein Ammoniumkation steht,

$R^1$ Wasserstoff, Niederalkyl, Phenyl, durch Niederalkyl substituiertes Phenyl, Pyrrolidyl oder die Gruppen

$-O-R^2$ oder $-O-\overset{\overset{O}{\|}}{C}-R^2$

in denen

$R^2$ für Niederalkyl steht,

bedeutet,

$R^3$ ein Rest aus der Gruppe

$-O^{\ominus}Me^{\oplus}$, $-O-R^4$, $-NH_2$, $-NHR^4$, $-NH-R^7-OH$,

$$-NH-\overset{O}{\overset{\|}{C}}-NH_2, \quad \text{oder} \quad -NH-R^7-\overset{O}{\overset{\|}{C}}-O^{\ominus}Me^{\oplus}$$

in der

$Me^{\oplus}$ die obengenannte Bedeutung hat,

$R^4$ Niederalkyl oder einen Etherrest ($C_1$ bis $C_6$ und 1 oder 2 Sauerstofatome) bedeutet,

$R^7$ Niederalkyl bedeutet,

n eine Zahl von 1 bis etwa 400 bedeutet, und

m im Bereich von etwa $10^3$ bis etwa $10^5$ liegt.

Insbesondere bevorzugt werden Copolymere auf Basis von Styrol und Maleinsäureanhydrid.

Besonders bevorzugt für die erfindungsgemäßen Abformmassen sind alternierende Copolymere mit einer Grenzviskositätszahl (gemessen in Dimethylformamid) im Bereich von 2 bis 6,5, bevorzugt 3 bis 5,5.

Besonders bevorzugte Copolymere für die erfindungsgemäßen Abformmassen sind dadurch gekennzeichnet, daß 20 bis 100 Mol-% des Copolymers, bevorzugt 40 bis 95 Mol-% Copolymer, als Alkali- oder Ammoniumsalz vorliegen.

Die erfindungsgemäßen Copolymere sind an sich bekannt (Elias Vohwinkel, Neue polymere Werkstoffe für die industrielle Anwendung, 2. Folge, Hanser-Verlag, Seiten 74 bis 78). Es ist auch bekannt, Copolymerisate von Maleinsäureanhydrid mit Styrol als Befestigungszement in der Zahnmedizin zu verwenden (DE 23 59 191, DE 26 20 955, GB 14 95 255, GB 15 04 708 und GB 15 07 981).

Die Copolymeren für die erfindungsgemäßen Abformmassen können hergestellt werden, indem man ein Olefin der Formel

$$R^1-CH=CH_2 \qquad (II)$$

in der

$R^1$ die obengenannte Bedeutung hat,

mit Maleinsäure anhydrid in Gegenwart eines Radikalstarters in einem Lösungsmittel polymerisiert und dann mit Verbindungen der Formel

$$R^3-H \qquad (III),$$

in der

$R^3$ die oben genannte Bedeutung hat,

umsetzt.

Als Olefine seien beispielsweise Ethylen, Isobutylen, Styrol und Vinylacetat genannt.

Als Radikalstarter können beispielsweise Peroxide, wie z.B. Dibenzoylperoxid, Dilauroylperoxid oder Azoverbindungen wie z.B. Azoisobuttersäuredinitril verwendet werden.

Als Lösungsmittel können Kohlenwasserstoffe und/oder Halogenkohlenwasserstoffe, wie z.B. Toluol oder 1,2-Dichlorethan, verwendet werden.

Die Herstellung der Copolymere wird im allgemeinen in an sich bekannter Weise durchgeführt (Braun, Cherdron, Kern, Praktikum der makromolekularen organischen Chemie, Verlag: Dr. A. Hüthig, Heidelberg, 175 (1966)).

Beispielsweise werden das Olefin und das Maleinsäureanhydrid in einem geeigneten Lösungsmittel gelöst. Nach Zugabe des Radikalstarters setzt die Polymerisation ein. Das Polymerisat fällt als feines Pulver aus und wird abgetrennt. Das erhaltene Maleinsäureanhydrid-Copolymer wird in an sich bekannter Weise durch Polymeranaloge-Umsetzung mit Verbindung der Formel (III) zu den erfindungsgemäßen Endprodukten umgesetzt.

Salze mehrwertiger Metalle für die erfindungsgemäßen Abformmassen sind im allgemeinen Salze 2- bis 6-wertiger Metalle. Bevorzugt werden Salze 2- bis 4-wertiger Metalle der zweiten oder dritten Hauptgruppe oder der Nebengruppen

des Periodensystems nach Mendelejew verwendet. Beispielsweise seien Salze des Calciums, Bariums, Aluminiums, Zinks, Mangans und/oder Eisens genannt.

Im allgemeinen enthalten die erfindungsgemäßen Abformmassen 3 bis 25 Gew.-Teile, bevorzugt 5 bis 15 Gew.-Teile, des Copolymerisats und 5 bis 35 Gew.-Teile, bevorzugt 10 bis 30 Gew.-Teile des Salzes mehrwertiger Metalle.

Die Salze mehrwertiger Metalle liegen in der Regel in Form der Salze anorganischer Säuren, vor. Bevorzugt werden schwerlösliche Salze mit einem Löslichkeitsprodukt von kleiner als $10^{-3}$, insbesondere bevorzugt $10^{-4}$ bis $10^{-10}$.

Beispielsweise seien die folgenden Salze mehrwertiger Metalle genannt: Calciumsulfat (gegebenenfalls als Mono- oder Dihydrat), Calciumsilikat, Bariumsulfat, Bariumsilikat, Zinkfluorid, Natriumaluminiumsilikat und Aluminiumsilikat.

Durch Zusatz der Salze mehrwertiger Metalle zu den Copolymerisaten entstehen vernetzte Copolymerisate. Solche Copolymerisate sind an sich bekannt.

In einer besonderen Ausführungsform enthalten die erfindungsgemäßen Abformmassen ein Copolymerisat der Formel (I), Salze mehrwertiger Metalle, Regulatoren und Füll-, Zuschlag-, Farb-, Geruch- und/oder Geschmackstoffe.

Regulatoren für die erfindungsgemäßen Abformmassen können beispielsweise Ammonium- und Alkali-phosphate, -pyrophosphate, -metaphosphate, -polyphosphate, -metasilikate und/oder-Citrate sein. Selbstverständlich können auch verschiedene Regulatoren eingesetzt werden.

Füllstoffe für die erfindungsgemäßen Abformmassen können beispielsweise Diatomeenerde, Kieselerde, Talkum, Quarzmehl, Cristobalitmehl oder gefällte oder pyrogene Kieselsäure sein. Bevorzugte Füllstoffe sind beispielsweise Kieselgur- oder Cristobalitmehl.

Zuschlagstoffe für die erfindungsgemäßen Abformmassen können beispielsweise Natriumfluorid, Natriumhexafluorosilikat, Kaliumhexafluorotitanat, Kaliumhexafluorozirkonat, Magnesiumoxid und/oder Zinkoxid sein.

Farbstoffe sind an sich für Abformmassen bekannte wasserunlösliche, anorganische und/oder organische Farbpigmente.

Geschmack- und Geruchstoffe können natürlicher und synthetischer Herkunft sein. Beispielsweise sei Pfefferminzöle genannt.

Die erfindungsgemäßen Abformmassen werden hergestellt, indem man ein oder mehrere Copolymere mit der Struktureinheit

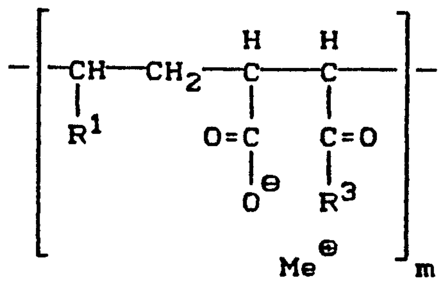 (I)

in der

$Me^{\oplus}$ für Wasserstoff, ein Alkali- oder ein Ammoniumkation steht,

$R^1$ Wasserstoff oder gegebenenfalls substituiertes Alkyl, Aryl, Heterocyclyl oder die Gruppen

$-O-R^2$ oder 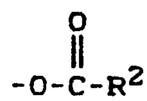

in denen

$R^2$ für Alkyl steht,

bedeutet,

$R^3$ ein Rest aus der Gruppe

$-O^{\ominus}Me^{\oplus}$, $-O-R^4$, $-NH_2$, $-NHR^4$, 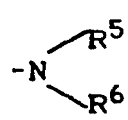 , $-NH-R^7-OH$,

$-NH-R^7-SO_3^{\ominus}Me^{\oplus}$, 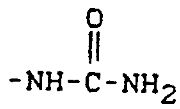, 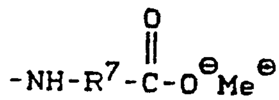 oder

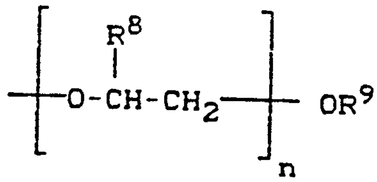

in denen

$Me^{\oplus}$ die obengenannte Bedeutung hat,

$R^4$,$R^5$,$R^6$ gleich oder verschieden sind und Alkyl oder einen Etherrest bedeuten,

wobei $R^5$ und $R^6$ über Alkylen oder einen zweibindigen Etherrest zu einem 5- oder 6-gliedrigen Ring verbunden sein können,

$R^7$ Alkylen bedeutet und

$R^8$ und $R^9$ gleich oder verschieden sind und Wasserstoff oder Niederalkyl bedeuten und

n eine Zahl von 1 bis etwa 2000 bedeutet, und

m im Bereich von etwa $10^2$ bis etwa $10^5$ liegt,

mit Salzen mehrwertiger Metalle, gegebenenfalls Regulatoren und gegebenenfalls Füll-, Zuschlag-, Farb-, Geruch- und/oder Geschmackstoffe vermischt.

Im allgemeinen setzt man 3 bis 20 Gew.-Teile, bevorzugt 5 bis 15 Gew.-Teile, des Copolymers, 5 bis 35 Gew.-Teile, bevorzugt 10 bis 30 Gew.-Teile eines Salzes mehrwertiger Metalle, 0,2 bis 10 Gew.-Teile, bevorzugt 0,5 bis 5 Gew.-Teile eines Regulators, 40 bis 90 Gew.-Teile, bevorzugt 50 bis 80 Gew.-Teile eines Füllstoffs, 0,5 bis 8 Gew.-Teile, bevorzugt 1 bis 5 Gew.-Teile eines Zuschlagstoffes, 0,1 bis 5 Gew.-Teile, bevorzugt 0,5 bis 3 Gew.-Teile eines Farbstoffs, 0,01 bis 1,5 Gew.-Teile, bevorzugt 0,05 bis 1,0 Gew.-Teile eines Geruchsstoffs und 0,5 bis 1,5 Gew.-Teile, bevorzugt 0,1 bis 1,0 Gew.-Teile, eines Geschmacksstoffs ein.

Die Bestandteile werden im allgemeinen in Mischern, z.B. einem Pflugscharmischer, zu den erfindungsgemäßen Abformmassen verarbeitet.

Für die Anwendung werden die erfindungsgemäßen Abdruckmassen in Form von Pulvern zubereitet. Sie werden im allgemeinen mit Wasser zu einer homogenen, hochviskosen und glatten Paste verarbeitet. Die Paste erstarrt nach kurzer Zeit, zu einem klebfreien, elastischen und reißfesten Gummi, der leicht von dem zu reproduzierenden Gegenstand gelöst werden kann.

Beispiel 1

In einem Pflugscharmischer wird eine Pulvermischung hergestellt durch Vermischen von 100 Teilen Polymer aus 1 Mol Styrol-Maleinsäureanhydrid-Copolymerisat (Molgewicht 550.000) und 2 Mol NaOH, 400 Teilen Kieselerde, 300 Teilen Cristobalitfeinstmehl, 160 Teilen Calciumsulfat-Dihydrat, 30 Teilen Tetranatriumdiphosphat und 10 Teilen anorganischer Farbstoff.

25 Teile der Pulvermischung und 15 Teile Wasser wurden innerhalb von 30 Sekunden zu einer homogenen, hochviskosen glatten Paste verarbeitet. 1 Minute 55 Sekunden nach Mischbeginn vernetzte die Paste zu einem klebfreien, elastischen und reißfesten Gummi.

Beispiel 2

Eine Pulvermischung wird in einem Pflugscharmischer hergestellt durch Mischen von 100 Teilen Polymer aus 1 Mol Styrol-Maleinsäureanhydrid-Copolymerisat (Molgewicht 460.000), 1/2 Mol NaOH und Wasser, 50 Teilen Calciumsilikat, 100 Teilen Aluminiumsilikat, 100 Teilen Calciumsulfat-Dihydrat, 600 Teilen Quarzfeinstmehl, 20 Teilen $Na_4P_2O_7$, 20 Teilen $K_2TiF_6$ und 10 Teilen anorganischen Farbstoff.

25 Teile der Pulvermischung und 25 Teile Wasser wurden innerhalb von 30 Sekunden zu einer hochviskosen, glatten Paste vermischt, welche 1 Minute und 40 Sekunden nach Mischbeginn zu einem klebfreien Gummi mit guter Elastizität und Reißfestigkeit vernetzte.

Beispiel 3

120 Teile Halbamid aus 1 Mol Poly(styrol-co-maleinsäureanhydrid) mit einem Molgewicht von 540.000 und 1/2 Mol Aminoessigsäure und 1 Mol NaOH, 550 Teilen Diatomeenerde, 120 Teilen Calciumsilikat, 180 Teilen Calciumsulfat-Dihydrat, 20 Teilen $Na_4P_2O_7$ und 10 Teilen anorganischem Farbstoff werden in einem Pflugscharmischer gemischt. 25 Teile der Pulvermischung und 35 Teile Wasser werden zu einer sehr glatten, mittelviskosen Paste verarbeitet, die 2 Minuten 10 Sekunden nach Mischbeginn zu einem elastischen, reißfesten Gummi vernetzte.

Beispiel 4

Eine Pulvermischung wird hergestellt in einem Pflugscharmischer durch Vermischen von 70 Teilen Halbamid aus Poly(styrol-co-maleinsäureanhydrid) mit einem Molgewicht von 400.000 und Harnstoff (K-Salz), 650 Teilen Cristobalitmehl, 100 Teilen ZnO, 150 Teilen $CaSO_4 \cdot 2H_2O$, 20 Teilen $Na_4P_2O_7$ und 10 Teilen anorganischem Farbstoff.

Eine hochviskose Paste wurde durch Vermischen von 25 Teilen der Pulvermischung und 15 Teilen Wasser erhalten, die nach 2 Minuten 40 Sekunden zu einem klebfreien, elastischen und reißfesten Gummi vernetzte.

Beispiel 5

120 Teile Polymer aus 1 Mol Ethylen-Maleinsäureanhydrid-Copolymerisat (MG 100.000) und 2 Mol NaOH, 100 Teile

Natriumaluminiumsilikat, 260 Teile Calciumsulfat-Dihydrat, 200 Teile Kieselerde, 300 Teile Quarzfeinstmehl, 10 Teile $KH_2PO_4$ und 10 Teile anorganischem Farbstoff werden in einem Pflugscharmischer zu einr Pulvermischung verarbeitet. Dia aus 25 Teilen Pulvermischung und 15 Teilen Wasser hergestellte, mittelviskose Paste vernetzte nach 3 Minuten 30 Sekunden zu einem elastischen, reißfesten Gummi.

Beispiel 6

Eine Pulvermischung wird hergestellt im Pflugscharmischer durch Vermischen von 150 Teilen Mononatriumsalz vom Poly-(isobutylen-co-maleinsäureanhydrid) mit Molgewicht 120.000, 200 Teilen Diatomeenerde, 300 Teilen Cristobalit-Feinstmehl, 200 Teilen Calciumsulfat-Hemihydrat, 100 Teilen Zinkoxid, 20 Teilen pyrogen hergestellte Kieselsäure mit 300 $m^2/g$ spezifischer Oberfläche nach BET, 20 Teilen $Na_4P_2O_7$ und 10 Teilen Farbstoff.

25 Teile Pulvermischung und 20 Teile Wasser ergeben durch Mischen eine hochviskose Paste, die nach 2 Minuten 40 Sekunden zu einem elastischen reißfesten Gummi vernetzte.

Beispiel 7

150 Teile des Dinatriumsalzes vom Poly(vinylacetat-co-maleinsäureanhydrid) mit einem Molgewicht von 80.000, 520 Teile Quarzfeinstmehl, 10 Teile Aluminiumsilikat, 100 Teile $CaSO_4 \cdot 2H_2O$, 100 Teile ZnO, 20 Teile $Na_4P_2O_7$ und 10 Teile anorganischem Farbstoff wurden in einem Pflugscharmischer zu einer Pulvermischung verarbeitet.

25 Teile dieser Pulvermischung und 15 Teile Wasser ergeben durch Mischen eine glatte hochviskose Paste, welche 1 Minute 50 Sekunden nach Mischbeginn zu einem elastischen, reißfesten Gummi vernetzte.

Beispiel 8

In einem Pflugscharmischer wird eine Pulvermischung hergestellt durch Vermischen von 100 Teilen Halbester aus 1 Mol Poly(styrol-co-maleinsäureanhydrid) mit einem Molgewicht von 480.000, 1 Mol Diethylenglykolmonomethylether und 1 Mol NaOH, 700 Teilen Cristobalitmehl, 150 Teilen $CaSO_4 \cdot 2H_2O$, 40 Teilen $Na_4P_2O_7$ und 10 Teilen anorganischem Farbstoff. Eine hochviskose Paste wird durch Vermischen von 25 Teilen der Pulvermischung und 20 Teilen Wasser erhalten, die nach 1 Minute 35 Sekunden zu einem elastischen, reißfesten Gummi vernetzt.

Patentansprüche

1. Abformmassen, enthaltend Copolymere mit der Struktureinheit

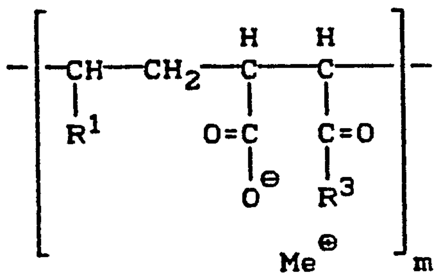

(I)

in der

$Me^{\ominus}$ für Wasserstoff, ein Alkali- oder ein Ammoniumkation steht,

$R^1$ Wasserstoff oder gegebenenfalls substituiertes Alkyl, Aryl, Heterocyclyl oder die Gruppen

$-O-R^2$ oder 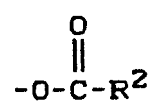

in denen

$R^2$ für Alkyl steht,

bedeutet,

$R^3$ ein Rest aus der Gruppe

$-O^{\ominus}Me^{\oplus}$, $-O-R^4$, $-NH_2$, $-NHR^4$, 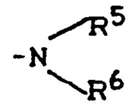, $-NH-R^7-OH$,

$-NH-R^7-SO_3^{\ominus}Me^{\oplus}$, 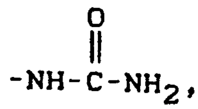, 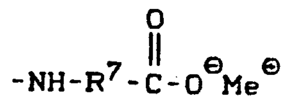 oder

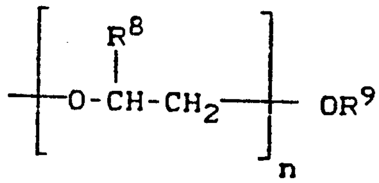

in denen

$Me^{\oplus}$ die obengenannte Bedeutung hat,

$R^4,R^5,R^6$ gleich oder verschieden sind und Alkyl oder einen Etherrest bedeutet,

wobei $R^5$ und $R^6$ über Alkylen oder einen zweibindigen Etherrest zu einem 5- oder 6-gliedrigen Ring verbunden sein können,

$R^7$ Alkylen bedeutet und

$R^8$ und $R^9$ gleich oder verschieden sind und Wasserstoff oder Niederalkyl bedeuten und

n eine Zahl von 1 bis etwa 2000 bedeutet, und

m im Bereich von etwa $10^2$ bis etwa $10^6$ liegt,

und Salze mehrwertiger Metalle.

2. Abformmassen nach Anspruch 1, enthaltend Copolymere worin

$Me^{\oplus}$ für Wasserstoff, ein Alkali- oder ein Ammoniumkation steht,

$R^1$ Wasserstoff oder gegebenenfalls substituiertes Alkyl (1 bis 8 Kohlenstoffatome), Aryl (6 bis 12 Kohlenstoffatome), Heterocyclyl (4 bis 10 Kohlenstoffatome und 1 oder 2 Sauerstoff und/oder Stickstoff) oder die Gruppen

$-O-R^2$ oder $-O-\overset{\overset{O}{\|}}{C}-R^2$

in denen

$R^2$ für Alkyl (1 bis 8 Kohlenstoffatome) steht,

bedeutet,

$R^3$ ein Rest aus der Gruppe

$-O^{\ominus}Me^{\oplus}$, $-O-R^4$, $-NH_2$, $-NHR^4$, 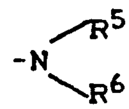 , $-NH-R^7-OH$,

$-NH-R^7-SO_3^{\ominus}Me^{\oplus}$, 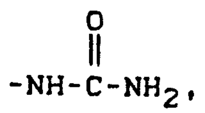, 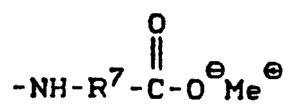 oder

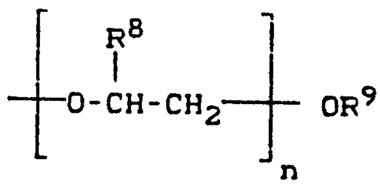

in der

$Me^{\oplus}$ die obengenannte Bedeutung hat,

$R^4, R^5, R^6$ gleich oder verschieden sind und Alkyl (1 bis 8 Kohlenstoffatome) oder einen Etherrest (1 bis 8 Kohlenstoffatome und 1 bis 3 Sauerstoffatome) bedeutet, wobei $R^5$ und $R^6$ über Alkylen oder einen zweibindigen Etherrest zu einem 5- oder 6-gliedrigen Ring verbunden sein können,

$R^7$ Alkylen mit 1 bis 8 Kohlenstoffatomen bedeutet,

$R^8$ und $R^9$ gleich oder verschieden sind und Wasserstoff oder Niederalkyl bedeuten und

n eine Zahl von 1 bis etwa 2000 bedeutet, und

m im Bereich von etwa $10^2$ bis etwa $10^6$ liegt,

und Salze mehrwertiger Metalle.

3. Abformmassen nach den Ansprüchen 1 und 2, enthaltend Copolymere, worin

$Me^{\oplus}$ für Wasserstoff, ein Alkali- oder ein Ammoniumkation steht,

$R^1$ Wasserstoff, Niederalkyl, Phenyl, durch Niederalkyl substituiertes Phenyl, Pyrrolidyl oder die Gruppen

$$-O-R^2 \quad \text{oder} \quad -O-\overset{\displaystyle O}{\overset{\|}{C}}-R^2$$

in denen

$R^2$ für Niederalkyl steht,

bedeutet,

$R^3$ ein Rest aus der Gruppe

$-O^{\ominus}Me^{\oplus}$, $-O-R^4$, $-NH_2$, $-NHR^4$, $-NH-R^7-OH$,

$$-NH-\overset{\overset{O}{\|}}{C}-NH_2 \quad \text{oder} \quad -NH-R^7-\overset{\overset{O}{\|}}{C}-O^{\ominus}Me^{\oplus}$$

in der

$Me^{\oplus}$ die obengenannte Bedeutung hat,

$R^4$ Niederalkyl oder einen Etherrest ($C_1$ bis $C_6$, 1 oder 2 Sauerstoffatome) bedeutet,

$R^7$ Niederalkylen bedeutet,

n eine Zahl von 1 bis etwa 400 bedeutet, und

m im Bereich von etwa $10^3$ bis etwa $10^5$ liegt,

und mehrwertige Metalle.

4. Abformmassen nach den Ansprüchen 1 bis 3, dadurch gekennzeichnet, daß ein alternierendes Copolymer mit einer Grenzviskositätszahl in Dimethylformamid im Bereich von 2 bis 6,5 eingesetzt wird.

5. Abformmassen nach den Ansprüchen 1 bis 4, dadurch gekennzeichnet, daß 20 bis 100 Mol-% des Copolymers als Alkali- oder Ammoniumsalz vorliegt.

6. Abformmassen nach den Ansprüchen 1 bis 5, dadurch gekennzeichnet, daß Salze zwei- bis sechswertiger Metalle eingesetzt werden.

7. Abformmassen nach den Ansprüchen 1 bis 6, dadurch gekennzeichnet, daß Salze zwei- bis vierwertiger Metalle der zweiten oder dritten Hauptgruppe oder der Nebengruppen des Periodensystems nach Mendelejew eingesetzt werden.

8. Abformmassen nach den Ansprüchen 1 bis 7, dadurch gekennzeichnet, daß Salze des Calciums, Bariums, Aluminiums, Zinks, Mangans und/oder Eisens eingesetzt werden.

9. Abformmassen nach den Ansprüchen 1 bis 8, dadurch gekennzeichnet, daß sie ein Copolymerisat, Salze mehrwertiger Metalle, Regulatoren und Füll-, Zuschlag-, Farb-, Geruch- und/oder Geschmackstoffe enthalten.

10. Abformmassen nach den Ansprüchen 1 bis 9, dadurch gekennzeichnet, daß als Regulatoren Ammonium- und Alkali-phosphate, -pyrophosphate, -metaphosphate, -polyphosphate, -metasilikate und/oder -citrate eingesetzt werden.

11. Abformmassen nach den Ansprüchen 1 bis 10, dadurch gekennzeichnet, daß als Füllstoffe Diatomeenerde, Kieselerde, Talkum, Quarzmehl, Cristobalitmehl oder Kieselsäuren eingesetzt werden.

12. Abformmassen nach den Ansprüchen 1 bis 11, dadurch gekennzeichnet, daß als Zuschlagstoffe Natriumfluorid, Natriumhexafluorosilikat, Kaliumhexafluorotitanat, Kaliumhexafluorozirkonat, Magnesiumoxid und/oder Zinkoxid eingesetzt werden.

13. Verwendung von Copolymeren mit der Struktureinheit

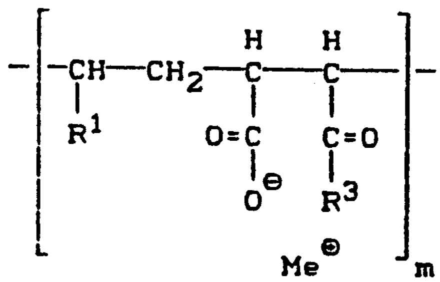

in der

$Me^{\ominus}$ für Wasserstoff, ein Alkali- oder ein Ammoniumkation steht,

$R^1$ Wasserstoff oder gegebenenfalls substituiertes Alkyl, Aryl, Heterocyclyl oder die Gruppen

$-O-R^2$ oder 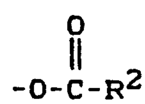

in denen

$R^2$ für Alkyl steht,

bedeutet,

$R^3$ ein Rest aus der Gruppe

$-O^{\ominus}Me^{\oplus}$, $-O-R^4$, $-NH_2$, $-NHR^4$, 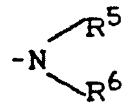 , $-NH-R^7-OH$,

$-NH-R^7-SO_3^{\ominus}Me^{\oplus}$, 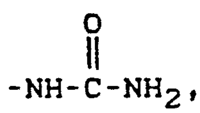, 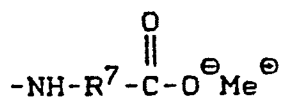 oder

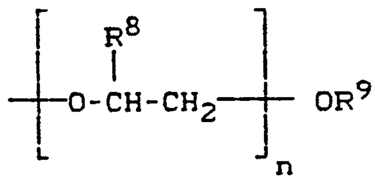

in denen

$Me^{\oplus}$ die obengenannte Bedeutung hat,

$R^4$,$R^5$,$R^6$ gleich oder verschieden sind und Alkyl oder einen Etherrest bedeuten,

0226924

wobei $R^5$ und $R^6$ über Alkylen oder einen zweibindigen Etherest zu einem 5- oder 6-gliedrigen Ring verbunden sein können,

$R^7$ Alkylen bedeutet und

$R^8$ und $R^9$ gleich oder verschieden sind und Wasserstoff oder Niederalkyl bedeuten und

n eine Zahl von 1 bis etwa 2000 bedeutet und

m im Bereich von etwa $10^4$ bis etwa $10^6$ liegt,

und Salze mehrwertiger Metalle

als Abformmassen.

14. Verwendung nach Anspruch 13 als Abformmasse im Dentalbereich.

15. Verfahren zur Herstellung von Abformmassen, dadurch gekennzeichnet, daß man ein oder mehrere Copolymere mit der Struktureinheit der Formel

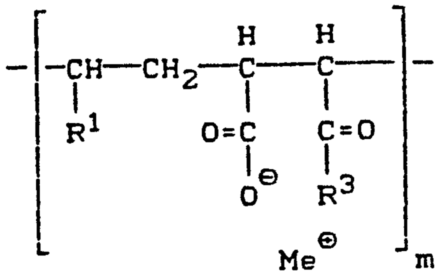

in der

$Me^{\oplus}$ für Wasserstoff, ein Alkali- oder ein Ammoniumkation steht,

$R^1$ Wasserstoff oder gegebenenfalls substituiertes Alkyl, Aryl, Heterocyclyl oder die Gruppen

$-O-R^2$ oder 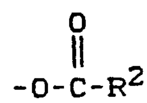

in denen

$R^2$ für Alkyl steht,

bedeutet,

$R^3$ ein Rest aus der Gruppe

$-O^{\ominus}Me^{\oplus}$, $-O-R^4$, $-NH_2$, $-NHR^4$, 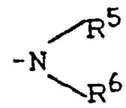 , $-NH-R^7-OH$,

$-NH-R^7-SO_3^{\ominus}Me^{\oplus}$, 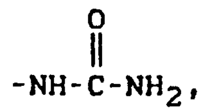, 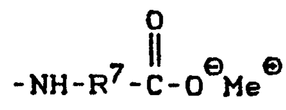 oder

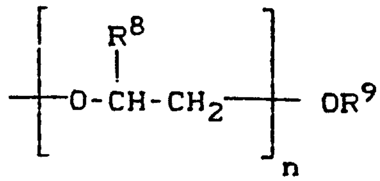

in denen

$Me^{\oplus}$ die obengenannte Bedeutung hat,

$R^4,R^5,R^6$ gleich oder verschieden sind und Alkyl oder einen Etherest bedeuten,

wobei $R^5$ und $R^6$ über Alkylen oder einen zweibindigen Etherrest zu einem 5- oder 6-gliedrigen Ring verbunden sein können,

$R^7$ Alkylen bedeutet und

$R^8$ und $R^9$ gleich oder verschieden sind und Wasserstoff oder Niederalkyl bedeuten und

n eine Zahl von 1 bis etwa 2000 bedeutet und

m im Bereich von etwa $10^2$ bis etwa $10^6$ liegt,

Salze mehrwertiger Metalle, gegebenenfalls Regulatoren und gegebenenfalls Füll-, Zuschlag-, Farb-, Geruch- und/oder Geschmackstoffe vermischt.

16. Verfahren nach Anspruch 15, dadurch gekennzeichnet, daß man 3 bis 20 Gew.-Teile eines Copolymers, 5 bis 35 Gew.-Teile eines Salzes mehrwertiger Metalle, 0,2 bis 10 Gew.-Teile eines Regulators, 40 bis 90 Gew.-Teile eines Füllstoffs, 0,5 bis 8 Gew.-Teile eines Zuschlagstoffes, 0,1 bis 5 Gew.-Teile eines Farbstoffs, 0,01 bis 1,0 Gew.-Teile eines Geruchsstoffs und 0,05 bis 1,5 Gew.-Teile eines Geschmacksstoffs einsetzt.

Europäisches Patentamt

# EUROPÄISCHER RECHERCHENBERICHT

0226924

Nummer der Anmeldung

EP 86117022.3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| A | EP - A2 - 0 149 354 (THE BRITISH PETROLEUM COMPANY)<br>* Zusammenfassung; Patentansprüche 1,10 * | 1,3,9,13-16 | A 61 K 6/10<br>C 08 L 35/00 |
| A | GB - A - 2 027 035 (REVERTEX LTD)<br>* Zusammenfassung; Patentansprüche 1,8,9,11,14 * | 1,3,9,11,13-16 | |
| A | CH - A - 283 908 (FARBENFABRIKEN BAYER)<br>* Beispiele 1-6,8; Seite 2, Zeilen 4-24 * | 1,3,9,11,13-16 | |
| D,A | DE - A1 - 2 620 955 (SMITH & NEPHEW RESEARCH LTD)<br>* Patentansprüche 1,20-26,31,44-49; Seiten 11-13 * | 1,3,9,11-16 | RECHERCHIERTE SACHGEBIETE (Int Cl 4)<br>A 61 K 6/00<br>B 22 C 1/00<br>B 22 C 7/00<br>C 08 F 8/00<br>C 08 F 210/00<br>C 08 F 212/00<br>C 08 F 222/00<br>C 08 L 23/00<br>C 08 L 31/00<br>C 08 L 35/00 |
| D,A | GB - A - 1 495 255 (NATIONAL RESEARCH DEVELOPMENT CORPORATION)<br>* Patentansprüche 1,7,8; Seite 1, Zeile 64 - Seite 2, Zeile 73 * | 1-4,9-16 | |
| D,A | GB - A - 1 504 708 (NATIONAL RESEARCH DEVELOPMENT CORPORATION)<br>* Patentansprüche 1-9,13-19; Seite 1, Zeile 39 - Seite 2, Zeile 30 * | 1,3,9,11-16 | |

Der vorliegende Recherchenbericht wurde für alle Patentansprüche erstellt

| Recherchenort | Abschlußdatum der Recherche | Prüfer |
|---|---|---|
| WIEN | 26-03-1987 | MAZZUCCO |

KATEGORIE DER GENANNTEN DOKUMENTEN
- X : von besonderer Bedeutung allein betrachtet
- Y : von besonderer Bedeutung in Verbindung mit einer anderen Veröffentlichung derselben Kategorie
- A : technologischer Hintergrund
- O : nichtschriftliche Offenbarung
- P : Zwischenliteratur
- T : der Erfindung zugrunde liegende Theorien oder Grundsätze

- E : älteres Patentdokument, das jedoch erst am oder nach dem Anmeldedatum veröffentlicht worden ist
- D : in der Anmeldung angeführtes Dokument
- L : aus andern Gründen angeführtes Dokument
- & : Mitglied der gleichen Patentfamilie, übereinstimmendes Dokument

EPA Form 1503 03 82

Europäisches Patentamt

# EUROPÄISCHER RECHERCHENBERICHT

Nummer der Anmeldung
EP 86117022.3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| D,A | GB - A - 1 507 981 (NATIONAL RESEARCH DEVELOPMENT CORPORATION) * Patentansprüche 1,8,13,20; Seite 2, Zeilen 6-20; Seite 3, Zeilen 18-23 * ---- | 1-3,9-16 | |
| | | | RECHERCHIERTE SACHGEBIETE (Int C 4) |

Der vorliegende Recherchenbericht wurde für alle Patentansprüche erstellt

| Recherchenort | Abschlußdatum der Recherche | Prüfer |
|---|---|---|
| WIEN | 26-03-1987 | MAZZUCCO |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82